# EUROPEAN PATENT APPLICATION

(11) **EP 1 704 876 A1**
(43) Date of publication of application: **27.09.2006**
(21) Application number: 05006474.0
(22) Date of filing: 24.03.2005
(51) Int. Cl.: A61L 15/44, A61L 27/34, A61L 27/54

(54) **Cosmetic treatment, device for performing said treatment and manufacturing method thereof**

(71) Applicant: NOLabs AB, 252 21 Helsingborg (SE)
(72) Inventor: Peters, Tor, 8703 Erlenbach (CH)
(74) Representative: Petri, Stellan

(57) **Abstract**

A method, and a device therefor, are provided that allow for cosmetic treatment of cosmetic disorders, caused by chronological age, environmental factors, changes in physiological functions of skin, such as psoriasis, dermatitis, acne, cellulites, and viral and/or bacteriological attacks, for example herpes, such as Herpes Simplex Virus type 1 (HSV-1), Herpes Simplex Virus type 2 (HSV-2), Epstein Barr Virus (EBV), CytoMegaloVirus (CMV), Varicella Zoster Virus (VZV), human herpes virus 6 (exanthum *subitum* and *roseola infantum),* human herpes virus 8 (HHV-8), caposis sarcoma, probably caused by HHV-8, warts, such as *verruca vulgaris, verruca planae*, *verruca seborroica,* filiform warts, mosaic warts, etc., caused by virus, and molluscs, caused by poxvirus. The device comprises a nitric oxide (NO) eluting polymer arranged to contact the area to be cosmetically treated, such that a cosmetic dose of nitric oxide is eluted from said nitric oxide eluting polymer to said area.

## Description

### Field of the Invention

This invention pertains in general to the field of cosmetic treatment, involving the use of nitric oxide (NO). More particularly the invention relates to a device for performing said treatment, and a process for manufacturing of said device, involving the use of nitric oxide (NO) for cosmetic purposes.

### Background of the Invention

In the society of today there is an increasing demand for products that will improve the physiological visual appearance of human beings.

Chronological age, environmental factors, changes in physiological functions of skin, psoriasis, dermatitis, cellulites, viral and/or bacteriological attacks, are some factors that affect the appearance of human beings in a cosmetically undesirable way.

Many of the alterations mentioned above are caused by changes in the outer epidermal layer of the skin, while others are caused by changes in the lower part of dermis. For instance, chronological age and extensive exposure to environmental factors, such as sun radiation, affect dermis in such way that dermis undergoes structural and functional changes, which result in many of the characteristics of aged skin, such as loss of elasticity, formation of wrinkles, loss of water-holding capacity, uneven distribution of fat, cellulites and sagging.

One thing that these factors have in common is that they are obtained by the loss of blood perfusion in the affected tissues.

Viral and bacteriological attacks may also result in impaired cosmetic appearance. Examples of such viral or bacteriological attack are herpes, such as Herpes Simplex Virus type 1 (HSV-1), Herpes Simplex Virus type 2 (HSV-2), Epstein Barr Virus (EBV), CytoMegaloVirus (CMV), Varicella Zoster Virus (VZV), human herpes virus 6 *(exanthum subitum* and *roseola infantum),* human herpes virus 8 (HHV-8), caposis sarcoma, probably caused by HHV-8, warts, such as *verruca vulgaris, verruca planae, verruca seborroica,* filiform warts, mosaic warts, etc., caused by virus, and molluscs, caused by poxvirus.

Psoriasis, such as *invers psoriasis, psoriasis guttata, psoriasis* pustulosa etc., is an inflammatory reaction in the skin, that may appear as a consequence of infection of *Streptococcus.* The disorder is not a self-healing disorder, and has to be treated, if the person suffering from psoriasis finds the disorder disfiguring or affecting his/her appearance in an undesirable way. Treatment of psoriasis is restricted to anti-inflammatory substances, such as glucocorticosteroids. This kind of treatment is often accompanied by adverse side effects, such as skin atrophy, telangiectasia, striae, hypertrichosis, rosacea, and dermatitis.

Dermatitis is another skin disorder that may disfigure a person, or affect the visual appearance of the person in a negative way.

The techniques according to the prior art, in respect of chronological age, environmental factors, changes in physiological functions of skin, include numerous of physiological, chemical, and mechanical methods, such as treatment with hydroxy acids, retinoids, barrier disrupters, tape stripping, solvent extraction etc. These methods present various drawbacks, such as irritation of the skin, skin toxicity, the requirement of high concentrations of expensive ingredients, pH values that are incompatible with the optimum pH value of the skin, long and cumbersome trials to establish whether or not a specific compound or composition is toxic or not, etc. Furthermore, the majority of the cosmetic methods according to the prior art induce invocation of damage of the skin, which results in the in set of repair mechanisms. Hence, there will be a period of time, such as weeks or months, during which the skin will remain irritated, and after which tolerance sets in and the irritations will diminish.

Up to this point there is no method, composition, compound etc., with the ability to simultaneously treat and prevent cosmetically undesirable disorders originating from both physiological factors, such as chronological age, environmental factors, changes in physiological functions of skin, such as psoriasis, dermatitis, cellulites, etc., and viral and bacteriological attacks.

Nitric oxide (NO) is a highly reactive molecule that is involved in many cell functions. In fact, nitric oxide plays a crucial role in the immune system and is utilized as an effector molecule by macrophages to protect itself against a number of pathogens, such as fungi, viruses, bacteria etc., and general microbial invasion. This improvement of healing is partly caused by NO inhibiting the activation or aggregation of blood platelets, and also by NO causing a reduction of inflammatory processes at the site of an implant.

NO is also known to have an anti-pathogenic, especially an anti-viral, effect, and furthermore NO has an anti-cancerous effect, as it is cytotoxic and cytostatic in suitable concentrations, i.e. it has among other effects tumoricidal and bacteriocidal effects. NO has for instance cytotoxic effects on human haematological malignant cells from patients with leukaemia or lymphoma, whereby NO may be used as a chemotherapeutic agent for treating such haematological disorders, even when the cells have become resistant to conventional anti-cancer drugs. This anti-pathogenic and anti-tumour effect of NO is taken advantage of by the present invention for cosmetic purposes, without having adverse effects.

However, due to the short half-life of NO, it has hitherto been very hard to treat viral, bacteria, virus, fungi or yeast infections with NO. This is because NO is actually toxic in high concentrations and has negative effects when applied in too large amounts to the body.

NO is actually also a vasodilator, and too large amounts of NO cause for instance a complete collapse of the circulatory system. On the other hand, NO has a very short half-life of fractions of a second up to a few seconds, once it is released. Hence, administration limitations due to short half-life and toxicity of NO have been limiting factors in the use of NO in the field of anti-pathogenic and anti-cancerous treatment so far.

In recent years research has been directed to polymers with the capability of releasing nitrogen oxide when getting in contact with water. Such polymers are for example polyalkyleneimines, such as L-PEI (Linear PolyEthyleneImine) and B-PEI (Branched PolyEthyleneImine), which polymers have the advantage of being biocompatible with natural products, after the release of nitrogen oxide.

Other example for NO eluting polymers are given in US-5,770,645, wherein polymers derivatized with at least one -NOₓ group per 1200 atomic mass unit of the polymer are disclosed, X being one or two. One example is an S-nitrosylated polymer and is prepared by reacting a polythiolated polymer with a nitrosylating agent under conditions suitable for nitrosylating free thiol groups.

Akron University has developed NO-eluting L-PEI molecule that can be nano-spun onto the surface of medical devices to be permanently implanted in the body, such as implanted grafts, showing significant improvement of the healing process and reduced inflammation when implanting such devices. According to US-6,737,447, a coating for medical devices provides nitric oxide delivery using nanofibers of linear poly(ethylenimine)-diazeniumdiolate. Linear poly(ethylenimine)diazeniumdiolate releases nitric oxide (NO) in a controlled manner to tissues and organs to aid the healing process and to prevent injury to tissues at risk of injury. Electrospun nano-fibers of linear poly(ethylenimine) diazeniumdiolate deliver therapeutic levels of NO for cosmetic purposes to the tissues surrounding a medical device while minimizing the alteration of the properties of the device. A nanofiber coating, because of the small size and large surface area per unit mass of the nanofibers, provides a much larger surface area per unit mass while minimizing changes in other properties of the device.

However, the disclosure is both silent concerning an improvement of present technology in respect of cosmetic treatment of physiologically factors, disorders, such as psoriasis and dermatitis, and viral and/or bacteriological attacks, by the use of NO.

Hence, an improved method and device for the treatment and/or prevention of cosmetic disorders, caused by chronological age, environmental factors, changes in physiological functions of skin, psoriasis, dermatitis, cellulites, viral and/or bacteriological attacks, which method and device do not develop resistance against the active pharmaceutical substance, and which preferably during the cosmetical treatment does not cause or causes minimal local skin irritation or contact allergic reactions, skin toxicity, the requirement of high concentrations of expensive ingredients, pH values that are incompatible with the optimum pH value of the skin, long and cumbersome trials to establish whether or not a specific compound or composition is toxic or not, skin atrophy, telangiectasia, striae, hypertrichosis, rosacea, dermatitis etc, would be advantageous, and in particular a method and device allowing for target improvement of the visual appearance would be advantageous.

### Summary of the Invention

Accordingly, the present invention preferably seeks to mitigate, alleviate or eliminate one or more of the above-identified deficiencies in the art and disadvantages singly or in any combination and solves at least the problems mentioned above, by providing a cosmetic treatment, a device for said cosmetic treatment, a manufacturing method for the latter and a use of nitric oxide according to the appended patent claims.

According to one aspect of the invention, a cosmetic treatment is provided that allows for target treatment of cosmetic disorders, caused by chronological age, environmental factors, changes in physiological functions of skin, such as psoriasis, dermatitis, cellulites, and viral and/or bacteriological attacks, for example herpes, such as Herpes Simplex Virus type 1 (HSV-1), Herpes Simplex Virus type 2 (HSV-2), Epstein Barr Virus (EBV), CytoMegaloVirus (CMV), Varicella Zoster Virus (VZV), human herpes virus 6 (exanthum *subitum* and *roseola infantum),* human herpes virus 8 (HHV-8), caposis sarcoma, probably caused by HHV-8, warts, such as *verruca vulgaris, verruca planae, verruca seborroica,* filiform warts, mosaic warts, etc., caused by virus, and molluscs, caused by poxvirus. The cosmetic method comprises an application of a nitric oxide (NO) eluting polymer arranged to contact the area to be treated, such that a cosmetic dose of nitric oxide is eluted from said nitric oxide eluting polymer to said area.

According to another aspect of the invention, a device is provided that allows for target treatment of cosmetic disorders, caused by chronological age, environmental factors, changes in physiological functions of skin, such as psoriasis, dermatitis, cellulites, and viral and/or bacteriological attacks, for example herpes, such as Herpes Simplex Virus type 1 (HSV-1), Herpes Simplex Virus type 2 (HSV-2), Epstein Barr Virus (EBV), CytoMegaloVirus (CMV), Varicella Zoster Virus (VZV), human herpes virus 6 *(exanthum subitum* and *roseola infantum),* human herpes virus 8 (HHV-8), caposis sarcoma, probably caused by HHV-8, warts, such as *verruca vulgaris, verruca planae, verruca seborroica,* filiform warts, mosaic warts, etc., caused by virus, and molluscs, caused by poxvirus. The device comprises a nitric oxide (NO) eluting polymer arranged to contact the area to be treated, such that a cosmetic dose of nitric oxide is eluted from said nitric oxide eluting polymer to said area.

According to another aspect of the invention, a manufacturing process for such a device is provided, wherein the process is a process for forming a device that allows for target treatment of cosmetic disorders, caused by chronological age, environmental factors, changes in physiological functions of skin, such as psoriasis, dermatitis, cellulites, and viral and/or bacteriological attacks, for example herpes, such as Herpes Simplex Virus type 1 (HSV-1), Herpes Simplex Virus type 2 (HSV-2), Epstein Barr Virus (EBV), CytoMegaloVirus (CMV), Varicella Zoster Virus (VZV), human herpes virus 6 (*exanthum* subitum and *roseola infantum),* human herpes virus 8 (HHV-8), caposis sarcoma, probably caused by HHV-8, warts, such as *verruca vulgaris, verruca planae, verruca seborroica,* filiform warts, mosaic warts, etc., caused by virus, and molluscs, caused by poxvirus. The process comprises selecting a plurality of nitric oxide eluting polymeric particles, such as nano fibres, fibres, nano particles, or microspeheres, and deploying said nitric oxide eluting particles in a condom/sheath or tape/coating to be comprised in said device. Alternatively the NO eluting particles are admixed to an ointment or cream.

The present invention has at least the advantage over the prior art that it provides target exposure of an area to be cosmetically treated to NO, whereby blood perfusion and vasodilatation are increased, whereby the supply of nutrients increase, simultaneously as an anti-viral, and an anti-microbial, effect is achievable.

### Brief Description of the Drawings

These and other aspects, features and advantages of which the invention is capable of will be apparent and elucidated from the following description of embodiments of the present invention, reference being made to the accompanying drawings, in which
Fig. 1 is a schematic illustration of a condom/sheath according to the invention,
Fig. 2 is a schematic illustration of a tape or coating according to the invention, and
Fig. 3 is a schematic illustration of a patch/pad according to the invention.

### Description of Embodiments

The following description focuses on embodiments of the present invention applicable to a device, in form of a condom/sheath, which allows for target treatment of cosmetic disorders, caused by chronological age, environmental factors, changes in physiological functions of skin, acne, psoriasis, dermatitis, cellulites, viral and/or bacteriological attacks, such as herpes, for example Herpes Simplex Virus type 1 (HSV-1), Herpes Simplex Virus type 2 (HSV-2), Epstein Barr Virus (EBV), CytoMegaloVirus (CMV), Varicella Zoster Virus (VZV), human herpes virus 6 *(exanthum subitum* and *roseola infantum),* human herpes virus 8 (HHV-8), caposis sarcoma, probably caused by HHV-8, warts, such as *verruca vulgaris, verruca planae, verruca seborroica,* filiform warts, mosaic warts, etc., caused by virus, and molluscs, caused by poxvirus.

With regard to nitric oxide (nitrogen monoxide, NO), its physiological and pharmacological roles have attracted much attention and thus have been studied. NO is synthesized from arginine as the substrate by nitric oxide synthase (NOS). NOS is classified into a constitutive enzyme, cNOS, which is present even in the normal state of a living body and an inducible enzyme, iNOS, which is produced in a large amount in response to a certain stimulus. It is known that, as compared with the concentration of NO produced by cNOS, the concentration of NO produced by iNOS is 2 to 3 orders higher, and that iNOS produces an extremely large amount of NO.

In the case of the generation of a large amount of NO as in the case of the production by iNOS, it is known that NO reacts with active oxygen to attack exogenous microorganisms and cancer cells, but also to cause inflammation and tissue injury. On the other hand, in the case of the generation of a small amount of NO as in the case of the production by cNOS, it is considered that NO takes charge of various protective actions for a living body through cyclic GMP (cGMP), such as vasodilator action, improvement of the blood circulation, antiplatelet-aggregating action, antibacterial action, anticancer action, acceleration of the absorption at the digestive tract, renal function regulation, neurotransmitting action, erection (reproduction), learning, appetite, and the like. Heretofore, inhibitors of the enzymatic activity of NOS have been examined for the purpose of preventing inflammation and tissue injury, which are considered to be attributable to NO generated in a large amount in a living body. However, the promotion of the enzymatic activity (or expressed amount) of NOS (in particular, cNOS) has not been examined for the purpose of exhibiting various protective actions for a living body by promoting the enzymatic activity of NOS and producing NO appropriately.

In recent years research has been directed to polymers with the capability of releasing nitrogen oxide when getting in contact with water. Such polymers are for example polyalkyleneimines, such as L-PEI (Linear PolyEthyleneImine) and B-PEI (Branched PolyEthyleneImine), which polymers have the advantage of being biocompatible, after the release of nitrogen oxide. Another advantage is that NO is released without any secondary products that could lead to undesired side effects. NO is released without by-products or breakdown products.

The polymers according to the present invention may be manufactured by electro spinning. Electro spinning is a process by which a suspended polymer is charged. At a characteristic voltage a fine jet of polymer releases from the surface in response to the tensile forces generated by interaction by an applied electric field with the electrical charge carried by the jet. This process produces a bundle of polymer fibres, such as nano-fibres. This jet of polymer fibres may be directed to a surface to be treated.

Furthermore, US 6,382,526, US 6,520,425, and US 6,695,992 disclose processes and apparatuses for the production of such polymeric fibres. These techniques are generally based on gas stream spinning, also known within the fiber forming industry as air spinning, of liquids and/or solutions capable of forming fibers.

Other example for NO eluting polymers are given in US-5,770,645, wherein polymers derivatized with at least one -NOX group per 1200 atomic mass unit of the polymer are disclosed, X being one or two. One example is an S-nitrosylated polymer and is prepared by reacting a polythiolated polymer with a nitrosylating agent under conditions suitable for nitrosylating free thiol groups.

Akron University has developed NO-eluting L-PEI molecule that can be nano-spun onto the surface of permanently implanted medical devices, such as implanted grafts, showing significant improvement of the healing process and reduced inflammation when implanting such devices. According to US-6,737,447, a coating for medical devices provides nitric oxide delivery using nanofibers of linear poly(ethylenimine)-diazeniumdiolate. Linear poly(ethylenimine)diazeniumdiolate releases nitric oxide (NO) in a controlled manner.

In one embodiment of the present invention the device is in form of fibres, nano-particles, or micro-spheres of a NO eluting polymer, which fibres, nano-particles, or micro-spheres are be integrated in a gel, cream, or foam, that may either be in a smearing or compressed structure.

These fibres, nano-particles, or micro-spheres, may be formed from the NO-eluting polymers comprised in the present invention, for example polyalkyleneimines, such as L-PEI (Linear PolyEthyleneImine) and B-PEI (Branched PolyEthyleneImine), which polymers have the advantage of being biocompatible, after the release of nitrogen oxide. They may also be encapsulated in any suitable material, such as polyvinylacetates, polylacticacids, starch, cellulose, polyhydroxyalkanoates, polyesters, polycaprolactone, polyvinylalcohol, protein based plastics, gelatine, biogradable polymers, and other soluble plastics.

According to an embodiment, the device is in the form of a lipstick-like device, which makes the NO especially easily applied to the skin or lips. Alternatively the gel cream, or foam is in form of or a dermatological ointment, cream or lotion for easy application to the body, e.g. in form of a spray bottle or a tube for easy application.

The device according to the present invention is applied on the area to be treated, such as any part of the body in need of improved cosmetic appearance, such as the face, neck, shoulders, hands, arms, back, chest, stomach, bottom, thigh, genitals, lower leg, and/or foot. Some places on the human body are of special interest for a majority of the population, such as the face, for the treatment of cosmetic deficiencies caused by or related to herpes, acne, wrinkles, sagging, loss of elasticity, loss of water-holding capacity, uneven distribution of fat, and the thigh, for the treatment of cellulites. Although these body parts commonly attract the most interest of the population, the present invention is not in any way intended to be limited to these.

When the gel, cream, or foam according to the present invention has been applied an elution of NO is initiated by adding water in any possible way. This may for example be accomplished by applying a water soaked patch on said gel, gel, cream, or foam, or spraying or bathing said gel, cream, or foam with water.

The cosmetic effect is obtained, as the NO eluting polymer elutes NO on the area to be treated, by an increased blood perfusion and vasodilatation, whereby an increased supply of nutrients in the tissue of interest is achieved. The increased blood perfusion and vasodilatation may, in another embodiment of the present invention, result in an improved effect when combined with other skin care products. Thus, this synergistic effect is within the scope of the present invention.

The fibres, nano-particles, or micro-spheres may also be integrated in a hydrogel, which is mixed directly before use.

This embodiment has the advantage of being able to penetrate pockets and corners in the skin for closer elution of NO on the area to be treated.

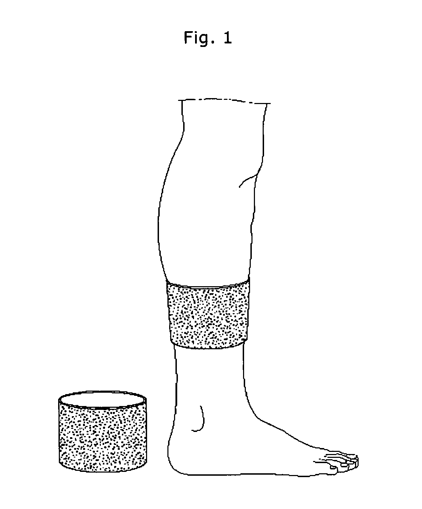

In another embodiment of the invention, according to Fig. 1, the device according to the present invention is in form of a latex or rubber condom/sheath, said condom/sheath being covered with nano-filament of any of the NO-eluting polymers according to above, such as polyalkyleneimines, such as L-PEI (Linear PolyEthyleneImine) and B-PEI (Branched PolyEthyleneImine), which polymers have the advantage of being biocompatible, after the release of nitrogen oxide.

In another embodiment of the present invention the condom/sheath is covered on the inside with nano-filament of L-PEI.

This condom/sheath may be in any suitable size, such as a suitable size for rolling said condom/sheath over the thigh, arm, neck, head, foot etc., to be treated. These sizes may for example vary from small, medium, and large sized condoms/sheaths in accordance with the different sizes, in respect of the different body parts, of persons in the population. The condom/sheath according to the invention may even have a size suitable for covering a foot, such as a sock, or a foot-condom/sheath, or other specific part of the body, to be able to obtain a cosmetic treatment. According to an embodiment, the condoms/sheaths are coated with NO eluting nano fibres. According to another embodiment the condoms/sheaths are made of, or comprise nanofilaments, e.g. made by electro or gas jet spinning. According to a further embodiment the condoms/sheaths comprises microspheres eluting NO in use. Preferably the three aforementioned embodiments employ L-PEI material loaded with NO. Activation on NO release may be done by e.g. foot sweat, water sprayed onto the condoms/sheaths immediately prior to use, or a water bag configured for releasing water upon activation, e.g. by pushing onto the bag thus bursting (see below).

When the NO-eluting condom/sheath according to certain embodiments of the present invention is treated with or gets in contact with the moisture, in form of secreted sweat, the NO-eluting condom/sheath starts to release NO to the area to be treated. Alternatively the device is moistured or wettened immediately prior to application or use for controlling or activating the NO release.

In another embodiment of the present invention the condom/sheath is covered on the inside with NO-eluting nano-particles, or micro-spheres, according to above.

When the nano-particles, or micro-spheres, according to this embodiment, gets in contact with the secreted moisture, in form of sweat, on the inside of the condom/sheath, they start to elute NO on the area to be treated.

In yet another embodiment of the present invention the condom/sheath contains a small water bag or sealed water sponge. This water bag or sealed water sponge is used to activate the elution of NO from the NO-eluting nano-particles, or micro-spheres. This water bag or sealed water sponge may be located in the tip of the condom/sheath according to the invention. Persons that not easily sweat may be helped by the use of this embodiment.

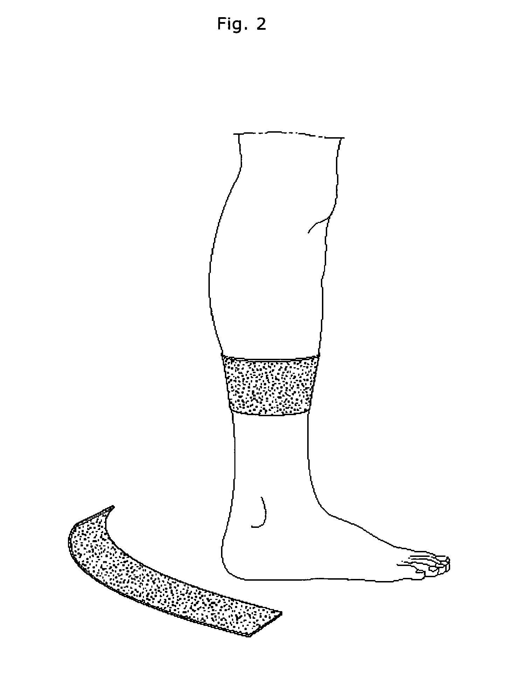

In still another embodiment of the device according to the present invention, it may be manufactured in the form of a polyurethane, or polyethylene, tape or coating, according to Fig. 2. This polyurethane tape or coating may easily be wrapped around, or applied on, the area to be cosmetically treated. At least the side facing the body part, may be covered with NO-eluting nano-particles, or micro-spheres, or nano-filament of NO-eluting L-PEI. When these particles or filaments get in contact with the moisture, in form of sweat, on the inside of the tape or coating, the elution of NO starts.

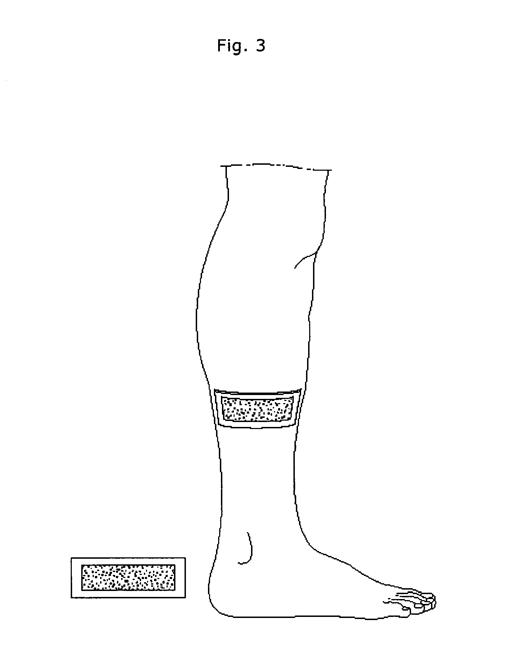

In another embodiment of the device according to the present invention, said device is in form of a patch/pad, according to Fig. 3, which patch/pad is suitable to be applied on the face, arm, hand, thigh, back, stomach, neck, to be cosmetically treated, or onto other areas that are difficult to cover with the condom/sheath according to the present invention. This patch/pad is attached by any suitable adhering means, such as materials that adhere to the skin.

Of course, in other embodiments of the invention, the patch/pad or tape/coating may be manufactured by any other suitable material, such as polyvinylacetates, polylacticacids, polyesters, polyamides, polyethers, polyurethanes, polycarbonates, cotton, starch, cellulose, polyhydroxyalkanoates, polyesters, polycaprolactone, polyvinylalcohol, polypropylene, polyacrylonitrile, polystyrene, poly(acrylic acid), protein based plastics, gelatine, biogradable polymers, and other soluble plastics. The NO-eluting polymer may be integrated in, spun together with, or spun on top of, any of these materials in all of the embodiments of the present invention.

In another embodiment these nano-particles, or micro-spheres, may be integrated in a soluble film that disintegrates on the inside of the condom/sheath or tape/coating according to the present invention, in order to elute NO at the area of interest when the soluble film gets in contact with the moisture, in form of sweat or from the water bag or sealed water sponge, on the area to be treated.

When placed on an area to be treated the device according to the present invention provides prevention and treatment of cosmetic disorders, caused by of chronological age, environmental factors, changes in physiological functions of skin, psoriasis, dermatitis, cellulites, viral and/or bacteriological attacks, for example herpes, such as Herpes Simplex Virus type 1 (HSV-1), Herpes Simplex Virus type 2 (HSV-2), Epstein Barr Virus (EBV), CytoMegaloVirus (CMV), Varicella Zoster Virus (VZV), human herpes virus 6 *(exanthum subitum* and *roseola infantum),* human herpes virus 8 (HHV-8), caposis sarcoma, probably caused by HHV-8, warts, such as *verruca vulgaris, verruca planae, verruca* seborroica, filiform warts, mosaic warts, etc., caused by virus, and molluscs, caused by poxvirus.

In another embodiment of the present invention the device according to the present invention only allows NO-elution in one direction. In this kind of embodiment one side of the condom/sheath or tape/coating is non-permeable to NO. This may be accomplished by applying a material on one side of the condom/sheath or tape/coating that is not permeable to NO. Such materials may be chosen from the group comprising common plastics, such as polyethylene, polyurethane etc. This embodiment is also easy to manufacture as the NO eluting polymer, e.g. L-PEI nano fibres may be electro or gas-jet spun onto the surface of a condom sheath of e.g. the mentioned plastics, latex, or cotton. In the case of a condom it may be rolled up, or a sheath may be turned outside in after manufacturing to protect the NO eluting polymer during packaging, transport and prior to use from external influences, being e.g. mechanical (abrasion of the polymer), chemical (moisture deactivating the device prior to use) etc.

In yet another embodiment of the present invention the NO-eluting device is acting as a booster for drug eluting patches, i.e. the device comprises additional agents e.g. pharmaceuticals, vitamins, nicotin, nitroglycerin, diclofenac etc. This embodiment presents a device with the advantage of combining two treatments, of significant value, in one treatment.

Hence, a synergetic effect may be achieved by such devices when NO that is eluted from the device. NO has a vasodilatory effect on the region where the device having the combination compound actuates. Vasodilated tissue is more susceptible to certain medications and thus more easily treated by the medical preparations and still NO has in addition to that the anti-inflamatory, anti-bacterial etc. effect. Hence, an unexpected surprisingly effective treatment is provided.

The cosmetic treatment with NO eluted from a polymer may also be combined with other agents in order to enhance the cosmetic treatment, for instance a desquamating agent, a moisturizer, a depigmenting or propigmenting agent, an anti-glycation agent, a 5.alpha.-reductase inhibitor, a lysyl and/or prolyl hydroxylase inhibitor, an agent for stimulating the synthesis of dermal or epidermal macromolecules and/or for preventing their degradation, an agent for stimulating keratinocyte proliferation and/or differentiation, a muscle relaxant, a further antimicrobial agent, a tensioning agent, an anti-pollution agent or a free-radical scavenger, or a combination thereof.

Preferably the NO eluting polymer is prepared and provided in a suitable form for topical application to keratin materials, including e.g. the skin, the eyelashes and the nails. The cosmetic use of a NO eluting polymer composition is for instance used for improving the appearance of such keratin materials. The cosmetic therapy includes a variety of fields, such as a use for preventing or treating wrinkles and fine lines and/or the loss of firmness, tonicity and/or elasticity of the skin and/or a dull complexion and/or dilation of the pores and/or skin or hair pigmentation disorders and/or skin dryness and/or hyperseborrhea and/or sensitive skin, and/or eventually hair loss and/or baldness. The composition or preparation comprising the NO eluting polymer should be provided in a biocompatible and/or a physiologically acceptable medium, suitable for the topical application to the keratine material mentioned above. Suitable forms include also moisturizing creams, anti-ageing creams, skin-peeling preparations. An alternative field of application is for cosmetically fading out pigmentary marks, in particular age marks. Moreover, cosmetic skin defects caused by acne may be cosmetically treated by topical treatment with a device according to the invention. Microbial colonization and inflammation may be removed in order to improve the appearance of acne exposed skin, for instance by the anti-inflammatory potency and/or the antibacterial potency without inducing bacterial resistance of NO.

The device according to the present invention elutes nitric oxide (NO) from said eluting polymer in a cosmetic dose, such as between 1 to 100 ppm, such as 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90 91, 92, 93, 94, 95, 96, 97, 98, 99, or 100 ppm.

In the embodiments of the present invention it may be suitable to control or regulate the time span of NO release from the device according to the invention. This may be accomplished by integrating other polymers or materials in said device. These polymers or materials may be chosen from any suitable material or polymer, such as polyvinylacetates, polylacticacids, starch, cellulose, polyhydroxyalkanoates, polyesters, polycaprolactone, polyvinylalcohol, protein based plastics, gelatine, biogradable polymers, and other soluble plastics.

The NO-eluting polymers in the devices according to the present invention may be combined with silver, such as hydroactivated silver. The integration of silver in the devices according to the present invention gives the healing process an extra boost. Preferably the silver is releasable from the devices in the form of silver ions.

The device according to the present invention may be manufactured by, for example electro spinning of L-PEI or other polymers comprising L-PEI or being arranged in combination with L-PEI. L-PEI is the charged at a characteristic voltage, and a fine jet of L-PEI releases as a bundle of L-PEI polymer fibres. This jet of polymer fibres may be directed to a surface to be treated. The surface to be treated may for example be any suitable material in respect of a device according to the present invention. The electro spun fibres of L-PEI then attach on said material and form a coating/layer of L-PEI on the device according to the invention.

It is of course possible to electro spin the other NO-eluting polymers, according to above, on the device according to the invention while still being inside the scope of the present invention.

In one embodiment the NO-eluting polymers according to the present invention are electro spun in such way that pure NO-eluting polymer fibres may be obtained.

It is also within the scope of the present invention to electro spin a NO-eluting polymer together with other suitable polymer/polymers.

Gas stream spinning, or air spinning, of said NO-eluting polymers onto the device according to the present invention is also within the scope of the present invention and has the advantage that application of the spinned particles is not dependent on an electric charge to be applied. Hence, gas jet or air spinning is advantageous in certain fields of application, e.g. when inflammable solvents are present.

The manufacturing process according to the present invention presents the advantages of large contact surface of the NO-eluting polymer fibres with the area to be treated, effective use of NO-eluting polymer, and a cost effective way of producing the device according to the present invention.

The invention may be implemented in any suitable form. The elements and components of the embodiments according to the invention may be physically, functionally, and logically implemented in any suitable way. Indeed, the functionality may be implemented in a single unit, in a plurality of units, or as part of other functional units.

Although the present invention has been described above with reference to specific embodiments, it is not intended to be limited to the specific form set forth herein. Rather, the invention is limited only by the accompanying claims and, other embodiments than the specific above are equally possible within the scope of these appended claims.

In the claims, the term "comprises/comprising" does not exclude the presence of other elements or steps. Furthermore, although individually listed, a plurality of means, elements or method steps may be implemented. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc do not preclude a plurality. Reference signs in the claims are provided merely as a clarifying example and shall not be construed as limiting the scope of the claims in any way.

## Claims

**1.** A device configured for cosmetic treatment of cosmetic disorders, including wrinkles, cellulites, psoriasis, dermatitis, and viral and/or bacteriological attacks, such as herpes, caposis sarcoma, warts, and/or mollusks, at a cosmetic treatment site of a body,
wherein
said device comprises a nitric oxide (NO) eluting polymer configured for eluting a cosmetic dosage of nitrogen oxide (NO) when used for said cosmetic treatment, and
wherein said device is configured for exposing said cosmetic treatment site of said cosmetic disorder of said body to said nitric oxide (NO) in use of said device being eluted from said polymer.

**2.** Device according to claim 1, wherein said polymer is selected from the group consisting of polyalkyleneimines, S-nitrosylated polymer, and poly(alkylenimine)diazeniumdiolates, or any combinations thereof.

**3.** Device according to claim 1, wherein said polymer is L-PEI (linear polyethyleneimine), loaded with nitric oxide (NO), arranged for release of the nitric oxide (NO) at said cosmetic treatment site of said cosmetic disorder at said body, for treatment of cosmetic disorders including wrinkles, cellulites, psoriasis, dermatitis, and viral and/or bacteriological attacks, such as herpes, caposis sarcoma, warts, and/or mollusks, thereby.

**4.** Device according to claim 1, wherein said device has a form selected from the group consisting of a condom/sheath, a sock, a patch/pad, and a tape/coating, adapted to be applied on or at said cosmetic treatment site of said cosmetic disorder on said body for treatment of cosmetic disorders, including wrinkles, cellulites, psoriasis, dermatitis, and viral and/or bacteriological attacks, such as herpes, caposis sarcoma, warts, and/or mollusks.

**5.** Device according to claim 4, wherein said condom/sheath, sock, patch/pad, and tape/coating is a polyurethane or polyethylene condom/sheath, sock, patch/pad, or tape/coating, including said nitric oxide (NO) eluting polymer configured for in use eluting said nitric oxide (NO) to said cosmetic treatment site of said cosmetic disorder of a body for treatment of said cosmetic disorders, including wrinkles, acne, cellulites, psoriasis, dermatitis, and viral and/or bacteriological attacks, such as herpes, caposis sarcoma, warts, and/or mollusks.

**6.** Device according to any of claims 1 to 5, including a water bag or sealed water sponge, configured for releasing water therefrom, when activated, to said device, and wherein said polymer is activateable to elute nitric oxide (NO) upon contact with said water.

**7.** Device according to claim 1, wherein said device is partly disintegrable when subjected to moisture or water.

**8.** Device according to claim 1, wherein said polymer comprises silver, configured for cosmetic treatment of said site of said cosmetic disorder on the body.

**9.** Device according to claim 1, wherein said polymer is comprised in the device in form of fibers, nano-particles or micro-spheres.

**10.** Device according to claim 9, wherein said fibres, nano-particles, or micro-spheres, are in the form of a gel, cream, foam, or hydrogel, or combinations thereof.

**11.** Device according to claim 9, wherein said nano-particles, or micro-spheres, are integrated with, preferably encapsulated in, a material, selected from the group consisting of polyvinylacetates, polylacticacids, starch, cellulose, cotton, polyhydroxyalkanoates, polyesters, polycaprolactone, polyvinylalcohol, protein based plastics, and/or gelatine, or combinations thereof.

**12.** Device according to claim 9 or 10, wherein said device is a lipstick-like device or a dermatological ointment, cream or lotion.

**13.** A manufacturing process for a device configured for cosmetic treatment of cosmetic disorders, infections, including including wrinkles, cellulites, psoriasis, dermatitis, and viral and/or bacteriological attacks, such as herpes, caposis sarcoma, warts, and/or mollusks, according to claim 1, comprising:
selecting a plurality of nitric oxide eluting polymeric particles, preferably nano fibres, nano particles or micro spheres, and
deploying said nitric oxide eluting particles into a suitable form, or as a coating onto a carrier, to form said device,
wherein said deploying comprises electro, air, or gas stream spinning of said particles.

**13.** Use of a nitric oxide (NO) eluting polymer for the manufacture of a device for the cosmetic treatment of cosmetic disorders, including wrinkles, cellulites, psoriasis, dermatitis, and viral and/or bacteriological attacks, such as herpes, caposis sarcoma, warts, and/or mollusks,
wherein
nitric oxide is loaded to said device so said device elutes nitric oxide (NO) from said eluting polymer in a cosmetic dose when used at a site of cosmetic disorder on a body.

**15.** Use according to claim 13, wherein said cosmetic dose is 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90 91, 92, 93, 94, 95, 96, 97, 98, 99, or 100 ppm.

**16.** A method of cosmetically treating a cosmetic disorder, including wrinkles, cellulites, acne, psoriasis, dermatitis, and viral and/or bacteriological attacks, such as herpes, caposis sarcoma, warts, and/or mollusks, comprising
applying a device, that comprises a nitric oxide (NO) eluting polymer configured for eluting a cosmetic dosage of nitrogen oxide (NO) when used for said cosmetic treatment, and thereby
exposing said cosmetic treatment site of said cosmetic disorder on a body to said nitric oxide when said polymer in use elutes nitrogen oxide (NO) by eluting a cosmetic dose of nitric oxide from said nitric oxide eluting polymer to said treatment site.

**17.** The method according to claim 16, wherein said site of said cosmetic disorder is a selected body region including face, neck, shoulder, back, arm, hand, stomach, genital, thigh, leg, or foot, of a body, and wherein said method comprises applying a condom/sheath, sock, patch/pad, and/or tape/coating or gel, cream, foam, or hydrogel, or combinations thereof, to said selected body region of said body, for said exposure.

**18.** The method according to claim 16 or 17 comprising
enhancing the cosmetic effect of a cosmetic treatment substance comprised in said device by cosmetic dose of nitric oxide.
